(19) Europäisches Patentamt
European Patent Office
Office européen des brevets

(11) Veröffentlichungsnummer : **0 171 755**
**B1**

(12) # EUROPÄISCHE PATENTSCHRIFT

(45) Veröffentlichungstag der Patentschrift : **25.05.88**

(51) Int. Cl.⁴ : **C 07 C 69/54**, C 08 F 20/18// A61K6/08

(21) Anmeldenummer : **85109956.4**

(22) Anmeldetag : **07.08.85**

(54) **Ester von Vinylcarbonsäuren.**

(30) Priorität : **18.08.84 DE 3430446**

(43) Veröffentlichungstag der Anmeldung : **19.02.86 Patentblatt 86/08**

(45) Bekanntmachung des Hinweises auf die Patenterteilung : **25.05.88 Patentblatt 88/21**

(84) Benannte Vertragsstaaten : **DE FR GB**

(56) Entgegenhaltungen :
**EP-A- 0 027 857**
**EP-A- 0 052 233**
**EP-A- 0 096 196**
**DE-A- 3 149 198**
**FR-A- 1 363 955**
**US-A- 4 336 345**

(73) Patentinhaber : **Röhm GmbH**
**Kirschenallee Postfach 4242**
**D-6100 Darmstadt 1 (DE)**

(72) Erfinder : **Hohage, Heinz-Jürgen, Dr.**
**In der Wildnis 3**
**D-6109 Mühltal (DE)**

**EP 0 171 755 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

## Gebiet der Erfindung

Die Erfindung betrifft neue Ester von Vinylcarbonsäuren als Monomere, die in radikalischer Polymerisation polymerisiert bzw. mit anderen Monomeren copolymerisiert werden können.

## Stand der Technik

Der Technik steht eine große Anzahl radikalisch polymerisierbarer Monomerer zur Verfügung. Eine bedeutende Rolle spielen Derivate der Acryl- und der Methacrylsäure, insbesondere deren Ester. So wurde auf der Grundlage speziell des Methylmethacrylats eine eigene Klasse von Kunststoffen entwickelt, die in vielen Bereichen der Technik ausgedehnte Anwendung findet (vgl. R. Vieweg, F. Esser, Kunststoff-Handbuch, Band IX, « Polymethacrylate », C. Hanser Verlag, 1975). Nebem dem Methylmethacrylat dienen zahlreiche andere Acryl- bzw. Methacrylverbindungen als monomere Ausgangsverbindungen für Polymerisate und Copolymerisate (vgl. J. Brandrup & E.H. Immergut. Polymer Handbook. 2. Auflage. Wiley-Interscience. 1975). Durch die Wahl der Monomeren und des Polymerisationsmodus lassen sich die Eigenschaften der gebildeten Polymeren beeinflussen. Umgekehrt rufen bestimmte Anforderungsprofile für Kunststoffe den Wunsch nach neuen Monomeren hervor, die den Anforderungen besser gerecht werden als die bereits auf dem Markt befindlichen. Obwohl der Polymer-Fachmann das Verhalten und den Einfluß eines einzelnen Monomeren auf das Polymerisat mehr oder weniger korrekt abzuschätzen und teilweise auch zu berechnen vermag, sind vielleicht abgesehen von gewissen strikt homologen Verbindungen Voraussagen nach wie vor mit beträchtlichen Unsicherheiten behaftet.

## Aufgabe und Lösung

Ungeachtet der vielen bekannten Acrylat- bzw. Methacrylmonomeren, besteht ein Bedarf an weiteren Monomeren, die es gestatten, bestimmte Polymerisateigenschaften herbeizuführen bzw. diese Eigenschaften zu beeinflussen. So sind z. B. weitere Monomere erwünscht zur Verbesserung der Eigenschaften von Dentalmassen auf Acrylatbasis.

Es wurde nun gefunden, daß neue Ester der Acryl- bzw. der Methacrylsäure der Formel I

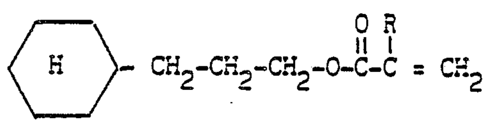

(I)

worin R für Wasserstoff oder einen Methylrest steht, die Anforderungen der Technik in besonderem Maße erfüllen.

Die neuen Ester der Formel I können in an sich bekannter Weise, z. B. durch Umsetzung aktivierter Derivate der Acryl- bzw. der Methacrylsäure der Formel II

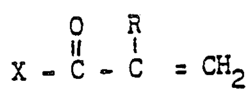

(II)

worin X für Chlor oder Brom oder für einen Rest

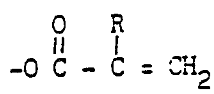

stehen, worin R die oben angegebene Bedeutung besitzt, mit 3-Cyclohexylpropanol, gegebenenfalls in Anwesenheit eines Säureakzeptors (vgl. US-PS 1 951 782) oder durch Umsetzung von Metall-, insbesondere Kalium- oder Silbersalzen der Acryl- bzw. Methacrylsäure mit 3-Cyclohexylpropylhalogeniden, insbesondere dem Chlorid oder Bromid oder mit Tosylaten, durch Veresterung der (Meth)acrylsäure mit 3-Cyclohexylpropanol, vorzugsweise unter Binden des gebildeten Wassers mittels dehydratisierenden Agentien oder vorzugsweise durch Umesterung der niederen Ester der Acryl- bzw. der Methacrylsäure der Formel III

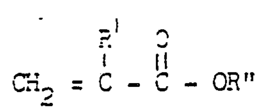

(III)

worin R′ für Methyl oder Wasserstoff und R″ für Methyl, Ethyl, Propyl oder Butyl steht, vorzugsweise in Gegenwart von Umesterungskatalysatoren hergestellt werden. (Vgl. H. Rauch-Puntigam. Th. Völker « Acryl- und Methacrylverbindungen », Springer Verlag, 1967, pp. 36-40. US-PS 2 138 763).

Als Umesterungskatalysatoren kommen z. B. basische Katalysatoren wie Alkalialkoholate (vgl. CH-PS 239 750), saure Katalysatoren wie p-Toluolsulfonsäure, Schwefelsäure (GB-PS 461 979, GB-PS 960 005) oder Orthotitansäureester (GB-PS 960 005 und GB-PS 962 928) in Frage.

Zweckmäßigerweise werden die Umsetzungen in Gegenwart von an sich bekannten Polymerisationsinhibitoren wie Chinone, Benzthiazin, Phenole, Methylenblau, aromatische Amine (Diphenylamin) oder Nitroverbindungen, gegebenenfalls auch Kupfer- oder Eisensalzen durchgeführt. Die anzuwendenden Inhibitormengen liegen meist in der Größenordnung von 0,01 bis 0,1 Gew.-% (bezogen auf eingesetzten Ester).

(Vgl. Ullmanns Encyklopädie der techn. Chemie, 4. Auflage, Bd. 15, pg. 256ff, Verlag Chemie, 1978). Genannt seien z. B. der Hydrochinonmonomethylether und das Hydrochinon.

Die folgenden Beispiele dienen zur Erläuterung der Erfindung. Die Verbindungen der Formel I sind farblose, destillierbare Flüssigkeiten. Sie eignen sich wie die bekannten Ester der (Meth)acrylsäure insbesondere zur Polymerisation oder Copolymerisation mit anderen, radikalisch polymerisierbaren Monomeren, insbesondere den Estern und Amiden der Acryl- bzw. der Methacrylsäure, Acryl- und Methacrylnitril, Styrol und dessen alkylierten Derivaten, Vinyläthern und -estern u. ä.

### Beispiel 1

In einem 4-l-Dreihalsrundkolben, ausgestattet mit Thermometer, Rückflußkühler mit Destillationsaufsatz und Rührer, werden 852 g (6 Mol) 3-Cyclohexylpropanol, 1 800 g (18 Mol) Methylmethacrylat, 0,18 g p-Methoxyphenol und 26 g Isopropyltitanat unter Einleiten von Luft zum Sieden erhitzt und solange ein Methanol/Methylmethacrylat-Azeotrop abgezogen, bis vollständiger Umsatz erreicht ist (ca. 3,5 h). Das überschüssige Methylmethacrylat wird im Rotationsverdampfer entfernt und der Rückstand destilliert. Man erhält das 3-Cyclohexylpropylmethacrylat in 91,5 % Ausbeute ; gaschromatographisch bestimmte Reinheit : > 98 %, Sdp. 0,8 mm : 88 °C, $n_D^{20}$ : 1,464.

### Beispiel 2

Völlig analog Beispiel 1 kann auch das 3-Cyclohexylpropylacrylat hergestellt werden, indem anstelle von Methylmethacrylat Äthylacrylat eingesetzt wird (Reaktionszeit 3 Std.).

| | |
|---|---|
| Ausbeute : | 81,5 % |
| Reinheit (GC) : | > 98 % |
| Sdp. 0,9 : | 85 °C |
| $n_D^{20}$ : | 1,4636 |
| $d_4^{20}$ : | 0,9480 |

### Beispiel 3

Dentalanwendung der Polymerisate.

Ein nach dem üblichen Perlpolymerisationsverfahren hergestelltes Perlpolymerisat aus 92 Gew.-Teilen Methylmethacrylat und 8 Gew.-Teilen 3-Cyclohexylpropylmethacrylat wird verwendet. Die zentrale Perlgröße beträgt $Z_2$ = 90 µm. Das Polymere wird nach dem üblichen Pulver-Flüssigkeitsverfahren (vgl. Ullmann's Encyclopädie der Techn. Chemie 4. Auflage, Band 10, pp. 8-9, Verlag Chemie) zu einer Zahnprothese verarbeitet. Der beim Anteigen mit 0,5 Teilen Methylmethacrylat erhaltene Teig ist sehr schnell (nach ca. 3 Minuten) klebfrei und läßt sich ca. 1 Stunde lang verarbeiten.

**Patentansprüche**

1. Ester von Vinylcarbonsäuren der Formel I

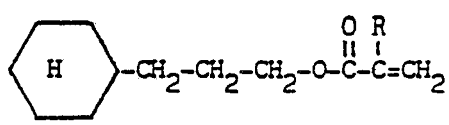

(I)

worin R für Wasserstoff oder Methyl steht.

2. Verfahren zur Herstellung von Polymerisaten durch radikalische Polymerisation von Monomeren, dadurch gekennzeichnet, daß man als Monomere die Ester von Vinylcarbonsäuren der Formel I

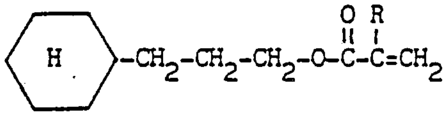 (I)

worin R für Wasserstoff oder Methyl steht, einsetzt.

## Claims

1. Esters of vinylcarboxylic acids of formula I

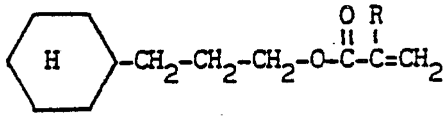 (I)

wherein R represents hydrogen or methyl.

2. Process for preparing polymers by radical polymerisation of monomers, characterised in that the monomers used are the esters of vinylcarboxylic acids of formula

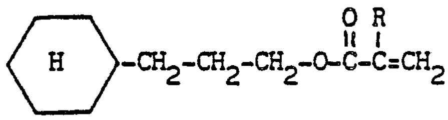 (I)

wherein R represents hydrogen or methyl.

## Revendications

1. Esters d'acides vinylcarboxyliques de formule I

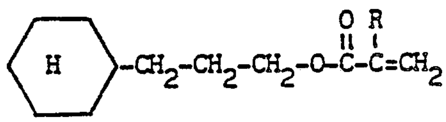 (I)

dans laquelle R est mis pour un atome d'hydrogène ou un radical méthyle.

2. Procédé de préparation de polymères par polymérisation radicalaire de monomères, caractérisé en ce qu'on utilise, comme monomères, les esters d'acides vinylcarboxyliques de formule I

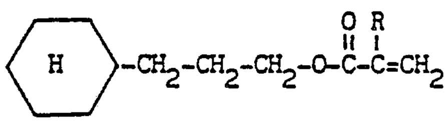 (I)

dans laquelle R est mis pour un atome d'hydrogène ou un radical méthyle.